## Europäisches Patentamt

## European Patent Office

## Office européen des brevets

(19)

(11) Veröffentlichungsnummer: **0 072 484**

**B1**

(12) **EUROPÄISCHE PATENTSCHRIFT**

(45) Veröffentlichungstag der Patentschrift:
**15.10.86**

(51) Int. Cl.⁴: **C 07 C 69/15**, C 07 C 67/54

(21) Anmeldenummer: **82106958.0**

(22) Anmeldetag: **02.08.82**

(54) **Verfahren zur Aufarbeitung von rohem, flüssigem Vinylacetat.**

(30) Priorität: **13.08.81 DE 3131981**

(43) Veröffentlichungstag der Anmeldung:
**23.02.83 Patentblatt 83/8**

(45) Bekanntmachung des Hinweises auf die Patenterteilung:
**15.10.86 Patentblatt 86/42**

(84) Benannte Vertragsstaaten:
**DE FR GB IT NL SE**

(56) Entgegenhaltungen:
**US-A-3 404 177**
**US-A-3 692 636**

(73) Patentinhaber: **BAYER AG, Konzernverwaltung RP Patentabteilung, D-5090 Leverkusen 1 Bayerwerk (DE)**

(72) Erfinder: **Wolf, Günter, Am Scherfenbrand 10, D-5090 Leverkusen 1 (DE)**
Erfinder: **Lauer, Hubert, Dr., Iltisweg 8, D-4047 Dormagen 12 (DE)**
Erfinder: **Schwerdtel, Wulf, Dr., Hegelstrasse 9, D-5090 Leverkusen (DE)**

EP 0 072 484 B1

**Beschreibung**

Die vorliegende Erfindung betrifft ein Verfahren zur Aufarbeitung von rohem, flüssigem Vinylacetat, das Essigsäure und Wasser, sowie Ethylacetat und gegebenenfalls noch weitere Verunreinigungen enthält.

Bei Verfahren zur Herstellung von Vinylacetat erhält man häufig ein flüssiges, rohes Vinylacetat, das im wesentlichen noch Essigsäure und Wasser, sowie Ethylacetat und gegebenenfalls noch weitere Verunreinigungen enthält. Ein derartiges Produkt kann z.B. bei der Herstellung von Vinylacetat durch Umsetzung von Ethylen mit Essigsäure und Sauerstoff in der Gasphase bei erhöhter Temperatur und normalem oder erhöhtem Druck in Gegenwart eines Katalysators erhalten werden, indem man die den Reaktor verlassenden Gase abkühlt und/oder mit Essigsäure wäscht. Eine derartige Herstellung von Vinylacetat wird im allgemeinen mit überschüssiger Essigsäure durchgeführt, wobei die nicht-umgesetzte Essigsäure im Kreislauf in die Vinylacetatherstellung zurückgeführt wird.

Eine Aufarbeitung von aus solchen Herstellungsverfahren zugänglichem, rohem, flüssigem Vinylacetat, das noch Essigsäure und Wasser, sowie Ethylacetat und gegebenenfalls noch weitere Verunreinigungen enthält, ist in der DE—AS 1 768 412 beschrieben. Bei diesem Verfahren wird das rohe Vinylacetat azeotrop destilliert, durch Rückführung des durch Kondensation und Phasentrennung des Kopfprodukts erhältlichen Vinylacetats die Hauptmenge des Wassers zusammen mit Vinylacetat über Kopf abgezogen und soviel Vinylacetat zurückgeführt, daß als Sumpfprodukt eine Essigsäure erhalten wird, die etwa 0,5 bis 6 Gew.-% Wasser enthält. Gegebenenfalls kann aus dieser Destillation ein flüssiger Seitenstrom entnommen werden, in dem Ethylacetat angereichert ist. Der Wassergehalt des gasförmigen Kopfproduktes aus der Destillation beträgt hierbei ca. 3,4 Gew.-% (s. die gemäß Beispiel 1 vorgenommene Nacharbeitung des Verfahrens der DE—AS 1 768 412). Das nach Kondensation und Phasentrennung des gasförmigen Kopfproduktes erhältliche Vinylacetat enthält etwa 200 bis 500 Gew.- ppm Ethylacetat.

Ein weiteres bekanntes Verfahren zur Aufarbeitung von rohem Vinylacetat wird in der DE—PS 1 282 013 und der DE—PS 1 668 063 beschrieben. Hierbei wird in einer ersten Destillationskolonne das Wasser als azeotropes Gemisch und die niedriger als Vinylacetat siedenden Nebenprodukte über Kopf destilliert und der praktisch wasserfreie Sumpf in einer zweiten Destillationskolonne destilliert, wobei man am Kopf der zweiten Destillationskolonne Vinylacetat, aus einer oder mehreren Anreicherungszonen zwischen Kopf und Sumpf mindestens den größten Teil der höher als Vinylacetat siedenden Nebenprodukte und unterhalb der untersten Anreicherungszone oder als Sumpf die Essigsäure und gegebenenfalls den Rest der Nebenprodukte eintnimmt. Das in der ersten Kolonne nach Kondensation und Phasentrennung anfallende Vinylacetat wird im allgemeinen vollständig in die erste Kolonne zurückgeführt und Vinylacetat nur vom Kopf der zweiten Kolonne entnommen. Angaben zum Wassergehalt des gasförmigen Kopfproduktes der ersten Kolonne werden nicht gemacht, der Ethylacetatgehalt im abgetrennten Vinylacetat beträgt etwa 1000 ppm.

In der DE—OS 2 943 985 wird ein Verfahren zur Abtrennung von Wasser aus Gemischen mit Vinylacetat und Essigsäure beschrieben, bei dem zwei unterschiedliche Arten rohen Vinylacetats an verschiedenen Stellen in eine Destillationskolonne gegeben werden. Das Kopfprodukt kann dann ca. 3 bis 5 Gew.-% Wasser enthalten. Ob Ethylacetat enthaltendes rohes Vinylacetat so aufgearbeitet werden kann und welche Gehalte an Ethylacetat dann im abgetrennten Vinylacetat vorkommen, ist dieser DE—OS nicht zu entnehmen.

Schließlich wird in der DE—AS 1 618 240 eine Abtrennung von Ethylacetat aus Vinylacetat beschrieben, bei der eine Extraktivdestillation mit Wasser als Schleppmittel durchgeführt wird. Das eingesetzte Vinylacetat enthält dabei jedoch keine nennenswerten Mengen an Wasser und/or Essigsäure.

Außerdem ist aus der US—PS 3,404,177 ein Verfahren zur Abtrennung von Vinylacetat, Wasser, Essigsäure und gegebenenfalls Acetaldehyd aus gasförmigen Reaktionsgemischen bekannt. Solche Reaktionsgemische enthalten auch noch Ethylen, Sauerstoff und $CO_2$. Ethylacetat und seine Abtrennung wird in dieser Schrift nicht erwähnt. Effekte, die mit einer Wassereinspeisung oder Azeotropbildung einhergehen, ebenfalls nicht.

Es wurde nun ein Verfahren zur Aufarbeitung von rohem, flüssigem Vinylacetat, das Essigsäure und Wasser, sowie Ethylacetat und gegebenenfalls weitere kleinere Mengen an Verunreinigungen enthält, durch Destillation, wobei man als Kopfprodukt ein im wesentlichen Vinylacetat und Wasser enthaltendes Gemisch und als Sumpfprodukt im wesentlichen Essigsäure erhält und einen Seitenstrom entnimmt, in dem Ethylacetat angereichert ist, und wobei man das Kopfprodukt kondensiert und nach Phasentrennung einen Teil der Vinylacetat-Phase als Rücklauf in die Destillation zurückführt gefunden, das dadurch gekennzeichnet ist, daß man in die Destillation oberhalb der Zugabestelle des rohen, flüssigen Vinylacetats 0,1 bis 5 Gew.-% Wasser, bezogen auf das eingesetzte Rohe, flüssige Vinylacetat, einleitet.

Zum Einsatz in das erfindungsgemäße Verfahren ist beispielsweise rohes, flüssiges Vinylacetat geeignet, das etwa 12 bis 30 Gew.-% Vinylacetat, etwa 3 bis 10 Gew.-% Wasser, etwa 0,05 bis 0,2 Gew.-% Ethylacetat und ergänzend auf 100 Gew.-% Essigsäure enthält. Weitere Verunreinigungen, beispielsweise Diacetate, Polymere, Acetaldehyd und/oder Methylacetat können ebenfalls in geringen Mengen, beispielsweise jeweils in Mengen von unter 0,5 Gew.-%, vorhanden sein. Solche Verunreinigungen können bei der Vinylacetat-Herstellung gebildet oder schon mit den Einsatzprodukten in die Vinylacetat-Herstellung eingebracht worden sein.

Ein für den Einsatz in das erfindungsgemäße Verfahren geeignetes rohes, flüssiges Vinylacetat kann

2

0 072 484

auf verschiedene Weise erhalten werden. Beispielsweise kann man in bekannter Weise Ethylen, Sauerstoff und Essigsäure in der Gasphase bei erhöhter Temperatur und normalem oder erhöhtem Druck an Edelmetall oder Edelmetallverbindungen enthaltenden Katalysatoren umsetzen und die aus dieser Umsetzung kommenden Gase abkühlen und/oder waschen, beispielsweise mit Essigsäure oder Essigsäure enthaltenden Waschflüssigkeiten, und das so erhältliche rohe, flüssige Vinylacetat in das erfindungsgemäße Verfahren einsetzen.

Das rohe, flüssige Vinylacetat wird in eine Destillationskolonne eingeleitet. Geeignete Destillationskolonnen sind beispielsweise solche, die 50 bis 90, vorzugsweise 55 bis 85 Böden enthalten und bei Normaldruck oder leicht erhöhtem Druck, beispielsweise bei 1 bis 5 bar absolut, vorzugsweise bei Normaldruck, betrieben werden. Die Zugabe des rohen, flüssigen Vinylacetats kann dabei beispielsweise an einer Stelle erfolgen, die im mittleren Bereich der Kolonne, vorzugsweise zwischen der Mitte und dem untersten Drittel der Kolonne, liegt. Die Kolonne wird so betrieben, daß man als Kopfprodukt ein im wesentlichen Vinylacetat und Wasser enthaltendes gasförmiges Gemisch und als Sumpfprodukt im wesentlichen Essigsäure erhält und einen Seitenstrom entnimmt, in dem Ethylacetat angereichert ist. Vorzugsweise betreibt man die Kolonne so, daß in der im Sumpf abgezogenen Essigsäure ein Wassergehalt von 0,5 bis 6 Gew.-% eingehalten und unterhalb des Zulaufs des Einsatzgemisches ein Seitenstrom entnommen wird, der beispielsweise bis zu 8 Gew.-% Ethylacetat neben schwankenden Mengen an Vinylacetat, Essigsäure und Wasser enthält. Dieser Seitenstrom kann flüssig oder gasförmig entnommen werden. Vorzugsweise wird dieser Seitenstrom in flüssiger Form entnommen. Das Kopfprodukt der Kolonne wird kondensiert und nach Phasentrennung ein Teil der Vinylacetat-Phase als Rücklauf in die Destillationskolonne zurückgeführt. Der Anteil des rückgeführten Vinylacetats kann dabei so reguliert werden, daß sich im Sumpf der Kolonne der gewünschte Wassergehalt, beispielsweise im Bereich von 0,5 bis 6 Gew.-%, einstellt. Hierfür ist im allgemeinen ein Rücklaufverhältnis (Verhältnis von zurückgeführtem zu entnommenem Vinylacetat) im Bereich von 3:1 bis 8:1 vorteilhaft. Das nichtzurückgeführte Vinylacetat wird abgetrennt und kann, falls erforderlich, noch weiter gereinigt werden. Das bei der Destillation anfallende Sumpfprodukt, das im allgemeinen zu über 90 Gew.-% Essigsäure enthält, kann in das Verfahren zur Herstellung von Vinylacetat zurückgeführt werden, gegebenenfalls nach Abtrennung von Verunreinigungen, z.B. Polymeren.

Bei dieser Arbeitsweise wird praktisch das gesamte mit dem rohen, flüssigen Vinylacetat eingebrachte Vinylacetat und ein Teil des mit dem rohen flüssigen Vinylacetat eingebrachten Wassers über Kopf genommen. Die bisher beschriebene Arbeitsweise, einschließlich der gegebenenfalls durchzuführenden weiteren Reinigung des abgetrennten Vinylacetats und der im Sumpf anfallenden Essigsäure ist beispielsweise aus der DE—AS 1 768 412 bekannt.

Die gemäß der vorliegenden Erfindung bei dieser Aufarbeitung von rohem, flüssigem Vinylacetat vorzunehmende Maßnahme besteht darin, daß man oberhalb der Zugabestelle des rohen, flüssigen Vinylacetats Wasser in einer Menge einleitet, die nicht größer ist als die Menge, die zum Erreichen eines maximalen Wassergehaltes in den die Destillation am Kopf verlassenden Gasen erforderlich ist.

Bereits geringe Mengen Wasser, beispielsweise 0,1 bis 1 Gew.-%, bezogen auf eingesetztes rohes, flüssiges Vinylacetat, die auf diese Weise eingeleitet werden, lassen den Wassergehalt in dem die Destillation am Kopf verlassenden Gas ansteigen und bewirken eine Verringerung des Ethylacetat-Gehaltes in dem aus dem Kopfprodukt der Destillation abgetrennten Vinylacetat. Wird mehr Wasser auf diese Weise zugegeben, so treten diese Effekte verstärkt auf, bis in den die Destillation am Kopf verlassenden Gasen ein maximaler Wassergehalt erreicht wird. Es ist bekannt, daß das Vinylacetat/Wasser-Azeotrop einen maximalen Gehalt von 7,3 Gew.-% Wasser aufweisen kann (s. Advances in Chemistry, Series 116, Azeotropic Data III, American Chemical Society, Washington D.C., 1973).

Dieser Wert kann aber bei der praktischen Durchführung des erfindungsgemäßen Verfahrens im allgemeinen nicht ganz erreicht werden. Die bei der praktischen Durchführung des erfindungsgemäßen Verfahrens maximal erreichbaren Wassergehalte in den die Destillation am Kopf verlassenden Gasen betragen etwa 6,8 Gew.-%. Vorzugsweise gibt man auf die erfindungsgemäße Weise gerade soviel Wasser zu, daß der Wassergehalt in dem die Destillation am Kopf verlassenden Gasen 6,7 bis 6,9 Gew.-% beträgt. Auf diese Weise werden die mit dem erfindungsgemäßen Verfahren erzielbaren Effekte optimal realisiert.

Wenn mehr Wasser zugegeben wird, als zum Erreichen eines maximalen Wassergehaltes in den die Destillation am Kopf verlassenden Gasen erforderlich ist, so erhöht sich der Wassergehalt in den die Destillation am Kopf verlassenden Gasen naturgemäß nicht weiter, die Verringerung des Ethylacetat-Gehaltes in dem aus dem Kopfprodukt der Destillation abgetrennten Vinylacetat bleibt erhalten und der Wassergehalt im Sumpf der Kolonne erhöht sich. Letzteres ist nicht erwünscht, da sonst der gesamte Wasserhaushalt im Vinylacetat-Herstellungsprozeß gestört würde.

Die erfindungsgemäß einzuleitende Wassermenge ist relativ gering. Sie beträgt 0,1 bis 5 Gew.-%, bezogen auf das eingesetzte rohe, flüssige Vinylacetat. Vorzugsweise beträgt diese Menge 0,5 bis 4 Gew.-%, besonders bevorzugt 0,8 bis 3 Gew.-%. Welche Wassermenge im konkreten Einzelfall notwendig ist, um in den die Destillation am Kopf verlassenden Gasen den höchstmöglichen Wassergehalt zu erreichen, kann gegebenenfalls durch einfache Versuche ermittelt werden. Diese Wassermenge hängt z.B. davon ab, wieviel Wasser und Vinylacetat das eingesetzte rohe Vinylacetat enthält.

Das erfindungsgemäß zuzusetzende Wasser kann in flüssiger Form oder als Wasserdampf in die Destillation eingeleitet werden. Die Wasserzugabe erfolgt oberhalb der Zugabestelle des rohen, flüssigen

3

Vinylacetats. Unterhalb des Zulaufs des rohen, flüssigen Vinylacetats oder mit diesem eingeleitetes Wasser verbessert nicht die Entwässerung in der Destillation und erniedrigt nicht den Ethylacetat-Gehalt in dem aus dem Kopfprodukt der Destillation abgetrennten Vinylacetat. Besonders vorteilhaft ist es, die erfindungsgemäße Einleitung von Wasser wenig oberhalb der Zugabestelle des rohen, flüssigen Vinylacetats vorzunehmen, beispielsweise 2 bis 5 Böden oberhalb der Zugabestelle des rohen, flüssigen Vinylacetats.

Die erfindungsgemäße Arbeitsweise hat folgende Vorteile:

— Die Entwässerung in der Destillation wird verbessert durch höher Wassergehalte in dem die Destillation am Kopf verlassenden Gasen. Das bedeutet, man kann entweder bei unverändertem Vinylacetat-Rücklauf im Sumpf einen bis zu 10 relativ % geringeren Wassergehalt als bisher erhalten, oder gleiche Wassergehalte im Sumpf wie bisher mit einem geringeren Vinylacetat-Rücklauf und/oder aus einem mehr Wasser enthaltenden rohen Vinylacetat und/oder in einer kürzeren Kolonne erzielen.
— Die Trennung Vinylacetat/Ethylacetat wird verbessert. Das bedeutet, man kann entweder bei unverändertem Vinylacetat-Rücklauf ein abgetrenntes Vinylacetat erhalten, das bis zu etwa 70 % weniger Ethylacetat als bisher enthält, oder einen gleichen Ethylacetat-Gehalt im abgetrennten Vinylacetat wie bisher bei einem geringeren Vinylacetat-Rücklauf und/oder aus einem mehr Ethylacetat enthaltenden rohen Vinylacetat und/oder in einer kürzeren Destillationskolonne erzielen.

Selbstverständlich kann man mit der vorliegenden Erfindung auch eine Kombination dieser Vorteile realisieren, beispielsweise einen verringerten Vinylacetat-Rücklauf und eine Erniedrigung des Wassergehaltes im Sumpf.

Besonders vorteilhaft ist es, daß die erfindungsgemäß erzielbaren Verbesserungen ohne Erhöhung des Aufwandes für die Destillation, z.B. in Bezug auf Bodenzahl, Rücklaufmengen und Energiebedarf, erreicht werden.

Es ist ausgesprochen überraschend, daß es durch die zusätzliche Einleitung von Wasser gemäß der vorliegenden Erfindung gelingt, die Entwässerungswirkung und die Vinylacetat/Ethylacetat-Trennung zu verbessern. Schon bei den bisher bekannten Aufarbeitungsmethoden für rohes, flüssiges Vinylacetat, bei denen Vinylacetat und Wasser über Kopf destilliert werden, ist ausreichend Wasser vorhanden, um theoretisch in den die Destillation am Kopf verlassenden Gasen einen Wassergehalt von ca. 7 Gew.-% erreichen zu können. Trotzdem ist es bisher in der Praxis nicht gelungen, solche Wassergehalte in den die Destillation am Kopf verlassenden Gasen zu erreichen. Beispielsweise ist in der DE—OS 2 943 985 angegeben, daß dort dieser Wassergehalt ca. 3 bis 5 Gew.-% beträgt, und bei der Arbeitsweise entsprechend der DE—AS 1 768 412 beträgt er ca. 3,4 Gew.-% (s. die gemäß Beispiel 1 vorgenommene Nacharbeitung des Verfahrens der DE—AS 1 768 412). Die Trennung Vinylacetat/Ethylacetat ist bekanntermaßen eine schwierige Aufgabe wegen der nahe beieinanderliegenden Siedepunkte beider Substanzen (Ethylacetat: 77°C, Vinylacetat: 73°C). Überraschend ist insbesondere auch, daß diese beiden Effekte, nämlich verbesserte Entwässerung und verbesserte Vinylacetat/Ethylacetat-Trennung, durch eine Maßnahme, nämlich die erfindungsgemäße Wasserzugabe, erzielt werden können.

Beispiele

Die wesentlichen Merkmale der im folgenden gegebenen Beispiele sind in Tabelle 1 zusammengefaßt.

Beispiel 1

(Vergleichsbeispiel, Arbeitsweise entsprechend DE—AS 1 768 412)

In eine Laborglockenbodenkolonne aus Glas mit 65 Böden wurden pro Stunde 1557 g eines Einsatzgemisches folgender Zusammensetzung auf den 25. Boden gegeben:

| | |
|---|---|
| Vinylacetat | 17,24 Gew.-% |
| Ethylacetat | 0,14 Gew.-% |
| Wasser | 3,83 Gew.-% |
| Essigsäure | 78,79 Gew.-% |

Das Kopfprodukt wurde kondensiert, auf 35°C abgekühlt und in einen Phasenabscheider geleitet. Von der Vinylacetatphase wurden 1056 g/h als Rücklauf auf den Kopf der Kolonne gepumpt, der Rest wurde entnommen. Die Zusammensetzung des abgetrennten Vinylacetats war wie folgt:

| | |
|---|---|
| Ethylacetat | 353 Gew. ppm |
| Wasser | 1,2 Gew.-% |
| Vinylacetat | Rest |

Die aus dem Phasenabscheider entnommene Wassermenge betrug 30,3 g/h, der Wassergehalt im Kopfdampf der Kolonne 3,37 Gew.-%.

Vom 8. Boden der Kolonne wurde ein flüssiger Seitenstrom in einer Menge von 29,5 g/h mit der Zusammensetzung

**4**

| Vinylacetat | 22,34 Gew.-% |
| Ethylacetat | 1,83 Gew.-% |
| Wasser | 4,52 Gew.-% |
| Essigsäure | 71,31 Gew.-% |

entnommen.

Die aus dem Sumpf der Kolonne entnommene Produktmenge betrug 1218 g/h und war wie folgt zusammengesetzt:

| Vinylacetat | 0,047 Gew.-% |
| Ethylacetat | 0,108 Gew.-% |
| Wasser | 2,07 Gew.-% |
| Essigsäure | Rest |

Beispiele 2 bis 5

In diesen Beispielen wurde die selbe Kolonne wie in Beispiel 1 unter den gleichen Bedingungen wie in Beispiel 1 betrieben. Zusätzlich wurde Wasser in die Kolonne gegeben und zwar in folgender Weise:

Beispiel 2: 15 g/h auf den 40. Boden der Kolonne
Beispiel 3: 30 g/h auf den 40. Boden der Kolonne
Beispiel 4: 40 g/h auf den 40. Boden der Kolonne
Beispiel 5: 40 g/h auf den 30. Boden der Kolonne

Die erzielten Ergebnisse sind in Tabelle 1 zusammengefaßt.

Daraus ist ersichtlich, daß durch die erfindungsgemäße Wasserzugabe der Wassergehalt im Sumpfprodukt erniedrigt wird und, daß die Ethylacetatgehalt im über Kopf abgezogenen Vinylacetat ganz erheblich absinkt. Es ist weiterhin ersichtlich, daß diese Effekte bei Erhöhung der Wassermenge und bei Absenkung des Zugabebodens ansteigen.

Ferner ist aus der Tabelle 1 ersichtlich, daß die aus dem Phasenabscheider entnommene Wassermenge immer größer ist als die Summe der beiden folgenden Wassermengen: 1) der Menge, die ohne erfindungsgemäße Wassereinspeisung entnommen wird (s. Vergleichsbeispiel 1) und 2) der Menge, die erfindungsgemäß zusätzlich eingespeist wird. Dies zeigt deutlich, daß nicht nur die erfindungsgemäß eingespeiste Wassermenge vollständig über Kopf genommen wird, sondern auch weitere, aus dem Einsatzgemisch stammende Wasseranteile.

Beispiel 6

Es wurde die selbe Kolonne wie in Beispiel 1 unter den gleichen Bedingungen wie in Beispiel 1 angegeben betrieben. Zusätzlich wurde Wasser in die Kolonne eingespeist und zwar 40 g/h auf den 30. Boden (wie in Beispiel 5).

In Abänderung zu den vorhergehenden Beispielen wurde der Rücklauf (s. Tabelle 1) soweit abgesenkt, daß das Sumpfprodukt der Kolonne praktisch den gleichen Wassergehalt aufwies wie in Beispiel 1 (ohne die erfindungsgemäße Wasserzugabe). Es ist ersichtlich, daß bei der erfindungsgemäßen Arbeitsweise auch bei vermindertem Rücklauf der Ethylacetatgehalt im Kopfprodukt ganz erheblich niedriger ist.

Beispiel 7
(zum Vergleich)

Es wurde verfahren wie in Beispiel 5, jedoch wurden anstelle von 40 g/h Wasser 50 g/h Wasser auf dem 30. Boden der Kolonne eingespeist. Dies ist mehr Wasser als zur Ausbildung eines Wassergehaltes des Kopfdampfes von 6,8 Gew.-% notwendig ist. Die zuviel eingespeiste Wassermenge findet sich im Sumpfprodukt wieder und erhöht dort den Wassergehalt. Auch bei dieser Arbeitsweise ist der Ethylacetatgehalt im Kopfprodukt sehr stark erniedrigt, jedoch ist diese Betriebsweise der Kolonne unvorteilhaft, da die im Sumpf anfallende Essigsäure nicht weit genug entwässert wird.

Beispiel 8
(zum Vergleich)

Es wurde die selbe Kolonne wie in Beispiel 1 unter den gleichen Bedingungen wie in Beispiel 1 betrieben.

Zusätzlich wurde Wasser in die Kolonne eingespeist, und zwar 40 g/h auf den 25. Boden, d.h. zusammen mit dem Vinylacetat-Zulaufgemisch. Aus Tabelle 1 ist ersichtlich, daß bei dieser Arbeitsweise der Wassergehalt im Sumpf stark ansteigt, der größte Teil des zusätzlich eingebrachten Wassers also über den Sumpf aus der Kolonne entnommen wird, während die zusätzlich über Kopf entnommene Wassermenge gering ist und der Ethylacetatgehält im Kopfprodukt nur unerheblich erniedrigt wird.

TABELLE 1

| Beispiel | Rücklauf g/h | Wasser-Einspeisung | | | Wassergehalt (Gew.-%) im | | Wasser aus phasenab-scheider g/h | Ethylacetat im organischen kopfprodukt gew. ppm |
| | | g/h | % *) | Boden | Sumpfprodukt | Kopfdampf | | |
| --- | --- | --- | --- | --- | --- | --- | --- | --- |
| 1**) | 1056 | — | — | — | 2,07 | 3,4 | 30,3 | 353 |
| 2 | 1056 | 15 | 0,96 | 40 | 1,89 | 4,6 | 49,4 | 272 |
| 3 | 1056 | 30 | 1,9 | 40 | 1,84 | 5,7 | 66,7 | 195 |
| 4 | 1056 | 40 | 2,5 | 40 | 1,80 | 6,4 | 73,2 | 150 |
| 5 | 1056 | 40 | 2,5 | 30 | 1,78 | 6,4 | 73,5 | 130 |
| 6 | 900 | 40 | 2,5 | 30 | 2,1 | 6,7 | 69,2 | 152 |
| 7**) | 1056 | 50 | 3,2 | 30 | 2,3 | 6,8 | 77,5 | 133 |
| 8**) | 1056 | 40 | 2,5 | 25 | 4,68 | 3,9 | 38,0 | 335 |

*) Gewichtsprozente bezogen auf eingesetzte Vinylacetatmischung
**) Die Beispiele 1, 7 und 8 sind nicht erfindungsgemäß

# 0 072 484

## Patentansprüche

1. Verfahren zur Aufarbeitung von rohem, flüssigem Vinylacetat, das Essigsäure und Wasser, sowie Ethylacetat und gegebenenfalls weitere kleinere Mengen anderer Verunreinigungen enthält, durch Destillation, wobei man als Kopfprodukt ein im wesentlichen Vinylacetat und Wasser enthaltendes Gemisch und als Sumpfprodukt im wesentlichen Essigsäure erhält und einen Seitenstrom entnimmt, in dem Ethylacetat angereichert ist und wobei man das Kopfprodukt kondensiert und nach Phasentrennung einen Teil der Vinylacetat-Phase als Rücklauf in die Destillation zurückführt, dadurch gekennzeichnet, daß man in die Destillation oberhalb der Zugabestelle des rohen, flüssigen Vinylacetats 0,1 bis 5 Gew.-% Wasser, bezogen auf das eingesetzte rohe, flüssige Vinylacetat, einleitet.

2. Verfahren nach Anspruch 1, dadurch gekennzeichnet, daß man rohes, flüssiges Vinylacetat aufarbeitet, das 12 bis 30 Gew.-% Vinylacetat, 3 bis 10 Gew.-% Wasser, 0,05 bis 0,2 Gew.-% Ethylacetat und ergänzend auf 100 Gew.-% Essigsäure enthält.

3. Verfahren nach Ansprüchen 1 und 2, dadurch gekennzeichnet, daß das rohe, flüssige Vinylacetat zusätzlich Diacetate, Polymere, Acetaldehyd und/oder Methylacetat, jeweils in Mengen unter 0,5 Gew.-% enthält.

4. Verfahren nach Ansprüche 1 bis 3, dadurch gekennzeichnet, daß man gerade soviel Wasser einleitet, daß der Wassergehalt in den die Destillation am Kopf verlassenden Gasen im Bereich 6,7 bis 6,9 Gew.-% liegt.

5. Verfahren nach Ansprüchen 1 bis 4, dadurch gekennzeichnet, daß man das Wasser in flüssigen Form einleitet.

6. Verfahren nach Ansprüchen 1 bis 4, dadurch gekennzeichnet, daß man das Wasser in Form von Wasserdampf einleitet.

7. Verfahren nach Ansprüchen 1 bis 6, dadurch gekennzeichnet, daß man das Wasser 2 bis 5 Böden oberhalb der Zugabestelle des rohen, flüssigen Vinylacetats einleitet.

## Revendications

1. Procédé pour purifier par distillation de l'acétate de vinyle liquide brut contenant de l'acide acétique et de l'eau ainsi que de l'acétate d'éthyle et le cas échéant des petites quantités d'autres impuretés, dans lequel on obtient en produit de tête un mélange contenant essentiellement de l'acétate de vinyle et de l'eau et en produit de pied de colonne essentiellement de l'acide acétique et on prélève un courant latéral dans lequel l'acétate d'éthyle est enrichi, on condense le produit de tête et, après séparation des phases, on recycle une partie de la phase acétate de vinyle en reflux dans la distillation, caractérisé en ce que, dans la distillation, on injecte au-dessus de l'endroit d'admission de l'acétate de vinyle liquide brut de 0,1 à 5 % en poids d'eau par rapport à l'acétate de vinyle liquide brut mis en oeuvre.

2. Procédé selon la revendication 1, caractérisé en ce que l'on purifie un acétate de vinyle liquide brut contenant 12 à 30 % en poids d'acétate de vinyle, 3 à 10 % en poids d'eau, 0,05 à 0,2 % en poids d'acétate d'éthyle, le complément à 100 % en poids consistant en acide acétique.

3. Procédé selon les revendications 1 et 2, caractérisé en ce que l'acétate de vinyle liquide brut contient en outre des diacétates, des polymères, de l'acétaldéhyde et/ou de l'acétate de méthyle, chacun en quantité inférieure à 0,5 % en poids.

4. Procédé selon les revendications 1 à 3, caractérisé en ce que l'on introduit l'eau en quantité juste suffisante pour que la teneur en humidité des gaz quittant la distillation en tête se situe dans l'intervalle de 6,7 à 6,9 % en poids.

5. Procédé selon les revendications 1 à 4, caractérisé en ce que l'on introduit l'eau à l'état liquide.

6. Procédé selon les revendications 1 à 4, caractérisé en ce que l'on introduit l'eau à l'état de vapeur.

7. Procédé selon les revendications 1 à 6, caractérisé en ce que l'on introduit l'eau à une distance de 2 à 5 plateaux au-dessus de l'endroit d'admission de l'acétate de vinyle liquide brut.

## Claims

1. Process for working up crude liquid vinyl acetate which contains acetic acid and water and also ethyl acetate and, possibly, further smaller amounts of other impurities, by distillation, a mixture essentially containing vinyl acetate and water being obtained as the top product and essentially acetic acid being obtained as the bottom product, and a sidestream, in which ethyl acetate is concentrated, being removed, and the top product being condensed and, after phase separation, part of the vinyl acetate phase being recycled as reflux into the distillation, characterised in that 0.1 to 5% by weight of water, based on the crude liquid vinyl acetate used, is introduced into the distillation, above the point of introduction of the crude liquid vinyl acetate.

2. Process according to Claim 1, characterised in that crude liquid vinyl acetate is worked up which contains 12 to 30% by weight of vinyl acetate, 3 to 10% by weight of water, 0.05 to 0.2% by weight of ethyl acetate, and acetic acid to make up to 100% by weight.

3. Process according to Claims 1 and 2, characterised in that the crude liquid vinyl acetate additionally

7

contains diacetates, polymers, acetaldehyde and/or methyl acetate, in each case in amounts of less than 0.5% by weight.

4. Process according to Claims 1 to 3, characterised in that only such an amount of water is introduced that the water content in the gases leaving the distillation at the top is in the range from 6.7 to 6.9% by weight.

5. Process according to Claims 1 to 4, characterised in that the water is introduced in liquid form.

6. Process according to Claims 1 to 4, characterised in that the water is introduced in the form of steam.

7. Process according to Claims 1 to 6, characterised in that the water is introduced 2 to 5 trays above the point of introduction of the crude liquid vinyl acetate.